(19) Europäisches Patentamt
European Patent Office
Office européen des brevets

(11) Publication number: 0 430 025 A2

(12) **EUROPEAN PATENT APPLICATION**

(21) Application number: 90122146.5

(51) Int. Cl.5: C07D 473/04, A61K 31/52

(22) Date of filing: 20.11.90

(30) Priority: 24.11.89 JP 303362/89
27.06.90 JP 166720/90

(43) Date of publication of application:
05.06.91 Bulletin 91/23

(84) Designated Contracting States:
AT BE CH DE DK ES FR GB IT LI NL SE
Bulletin

(71) Applicant: HOKURIKU PHARMACEUTICAL CO., LTD.
1-Chome, 3-14, Tatekawacho, Katsuyama-shi
Fukui-ken(JP)

(72) Inventor: Ito, Yasuo
11-14, Moto-machi 3-chome
Katsuyama-shi, Fukui-ken(JP)
Inventor: Kato, Hideo
5-8, Kentoku 3-chome
Fukui-shi, Fukui-ken(JP)
Inventor: Koshinaka, Eiichi
6-3, Asahi-cho 2-chome
Katsuyama-shi, Fukui-ken(JP)
Inventor: Yagi, Noriyuki
12-6-2, Inokeya
Katsuyama-shi, Fukui-ken(JP)
Inventor: Iwasaki, Nobuhiko
12-6-2, Inokeya
Katsuyama-shi, Fukui-ken(JP)

(74) Representative: Werner, Hans-Karsten, Dr. et al
Deichmannhaus am Hauptbahnhof
W-5000 Köln 1(DE)

(54) **Xanthine compound, method for preparing thereof, and a pharmaceutical composition comprising the same.**

(57) A novel xanthine compound having bronchodilating and brain function improving activity represented by the following formula (I) and (II):

EP 0 430 025 A2

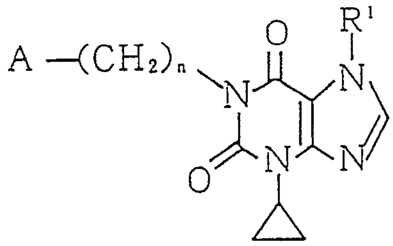

( I )

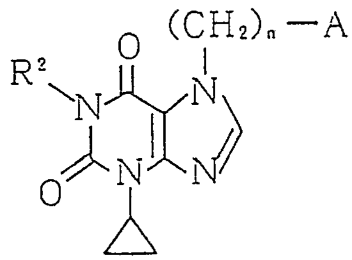

( II )

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms is disclosed. Also disclosed are a pharmacologically acceptable salt of the same, method for preparing the same, and a pharmaceutical composition for the treatment of respiratory tract disease or brain disfunction comprising the effective amount of same.

# XANTHINE COMPOUND, METHOD FOR PREPARING THEREOF, AND A PHARMACEUTICAL COMPOSITION COMPRISING THE SAME

## BACKGROUND OF THE INVENTION

### Field of the invention

The present invention relates to novel xanthine compounds and pharmacologically acceptable salts thereof which have a bronchodilating activity and improving activity for brain disfunction and are useful for the treatment of, for example, respiratory tract disease or brain disfunction, and to the method for preparing thereof.

The present invention also relates to a pharmaceutical composition comprising the effective amount of the same.

### Description of the Prior Art

Xanthine compound such as theophylline (The Merck Index, 11th edition, 9212), pentoxifylline (The Merck Index, 11th edition, 7092), and propentofylline (The Merck Index, 11th edition, 7822) have been widely used clinically for the treatment of respiratory tract disease or brain disfunction.

Chief clinical disadvantages of xanthine compound are severe adverse reactions frequently induced by the administration of these compounds. Examples of the adverse reaections are, for example, cardio excitatory activity such as, for example, cardiopalmus or tachycardia; central activity such as, for example, convulsion or headache; and gastrointestinal activity such as, for example, nausea or emesis. Therefore, clinical developments of a xanthine compound which eliminates most of these adverse reactions has been longed for from a clinical point of view.

In addition, a xanthine compound substituted with an oxo-alkyl group in the 1- or 7-position of the xanthine nucleus which is substituted with a cyclopropyl group in the 3-position of the nucleus has not been known to date.

## SUMMARY OF THE INVENTION

An object of the present invention is to provide a novel xanthine compound and a pharmacologically acceptable salt of the compound having an excellent bronchodilating activity and improving activity for brain disfunction.

Another object of the present invention is to provide a xanthine compound and a pharmacologically acceptable salt of the compound eliminating adverse reactions such as cardio excitatory acivity, central activity, or gastrointestinal activity.

A further object of the present invention is to provide a method for preparing the xanthine compound and a pharmaceutical composition comprising the same.

The inventors of the present invention have conducted various studies to achieve the foregoing objects and found that the objects can effectively attained by providing a novel xanthine compound of the present invention having an excellent bronchodilating activity and improving activity for brain disfunction. The compound of the present invention is useful for the clinical treatment of respiratory tract disease or brain disfunction since it eliminates most of possible adverse reactions such as cardio excitatory acivity.

Thus, in accordance with the above objects, the present invention provides a novel xanthine compound represented by the following formula (I) or (II):

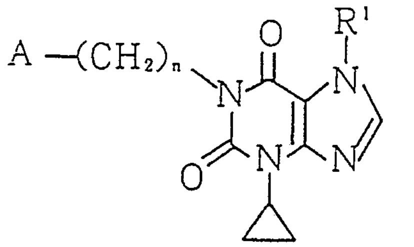

(I)

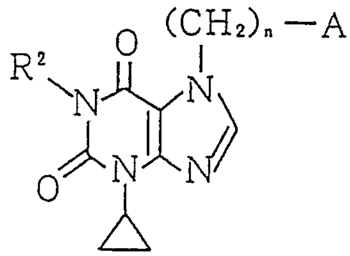

(II)

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms, and a pharmacologically acceptable salt of the above compound.

In accordance with another embodiment of the present invention, the present invention provides a process for preparing the xanthine compound represented by the above formula (I) or (II).

In accordance with yet another embodiment, the present invention provides a bronchodilating agent comprising the xanthine compound represented by the above formula (I) or (II), an agent for improving brain disfunction comprising the xanthine compound represented by the above formula (I) or (II), and a pharmaceutical composition for the treatment of respiratory tract disease or brain disfunction comprising the effective amount of the xanthine compound represented by the above formula (I) or (II) together with a pharmaceutically acceptable carrier or coating.

In accordance with a further embodiment, the present invention provides a method for treatment of respiratory tract disease or brain disfunction comprising the step of administering to a mammal an effective amount of the xanthine compound represented by the above formula (I), a pharmacologically acceptable salt of the compound, a bronchodilating agent or an agent for improving brain disfunction comprising the same, or a pharmaceutical composition comprising the same together with a pharmaceutically acceptable carrier or coating.

## DETAILED DESCRIPTION OF THE PREFERRED EMBODIMENTS

The present invention provides a novel xanthine compound represented by the following formula (I) or (II):

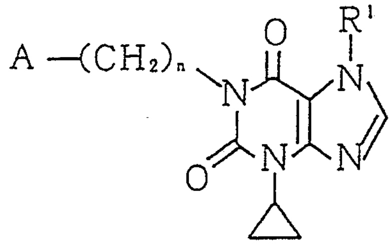

(I)

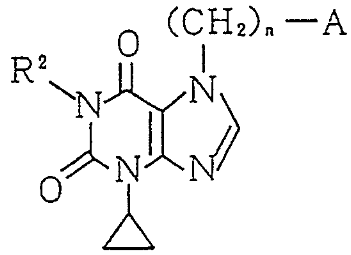

(II)

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms, and a pharmacologically acceptable salt of the above compound.

In the above formula (I) and (II), examples of the straight-or branched-chain alkyl group having 1 to 6 carbon atoms include, for example, methyl, ethyl, n-propyl, isopropyl, n-butyl, isobutyl, sec-butyl, tert-butyl, n-pentyl, 2-methylbutyl, and n-hexyl groups; examples of the cycloalkyl group having 3 to 6 carbon atoms include, for example, cyclopropyl, cyclobutyl, cyclopentyl, and cyclohexyl groups; examples of the alkyl group having 1 to 3 carbon atoms represented independently by $R^3$ and $R^4$ include, for example, methyl, ethyl, and n-propyl groups; and the polymethylene group having 2 to 4 carbon atoms represented by $R^3$ together with $R^4$ include, for example, ethylene and propylene groups.

Particularly preferred examples of the present invention include:

3-cyclopropyl-3,7-dihydro-7-methyl-1-(2-oxopropyl)-1H-purine-2,6-dione;
3-cyclopropyl-7-ethyl-3,7-dihydro-1-(2-oxopropyl)-1H-purine-2,6-dione;
3-cyclopropyl-3,7-dihydro-1-(2-oxopropyl)-7-n-propyl-1H-purine-2,6-dione;
3-cyclopropyl-3,7-dihydro-7-methyl-1-(3-oxobutyl)-1H-purine-2,6-dione;
3-cyclopropyl-7-ethyl-3,7-dihydro-1-(3-oxobutyl)-1H-purine-2,6-dione;
3-cyclopropyl-3,7-dihydro-1-(3-oxobutyl)-7-n-propyl-1H-purine-2,6-dione;
3-cyclopropyl-3,7-dihydro-7-methyl-1-(4-oxopentyl)-1H-purine-2,6-dione;
3-cyclopropyl-7-ethyl-3,7-dihydro-1-(4-oxopentyl)-1H-purine-2,6-dione;
3-cyclopropyl-3,7-dihydro-1-(4-oxopentyl)-7-n-propyl-1H-purine-2,6-dione;
3-cyclopropyl-3,7-dihydro-1-(5-oxohexyl)-1H-purine-2,6-dione;
3-cyclopropyl-3,7-dihydro-7-methyl-1-(5-oxohexyl)-1H-purine-2,6-dione;
3-cyclopropyl-7-ethyl-3,7-dihydro-1-(5-oxohexyl)-1H-purine-2,6-dione;
3-cyclopropyl-3,7-dihydro-1-(5-oxohexyl)-7-n-propyl-1H-purine-2,6-dione;
7-n-butyl-3-cyclopropyl-3,7-dihydro-1-(5-oxohexyl)-1H-purine-2,6-dione;
3-cyclopropyl-3,7-dihydro-1-(5-oxohexyl)-7-n-pentyl-1H-purine-2,6-dione;
3-cyclopropyl-7-n-hexyl-3,7-dihydro-1-(5-oxohexyl)-1H-purine-2-6-dione;
3-cyclopropyl-3,7-dihydro-7-(4-methoxybenzyl)-1-(5-oxohexyl)-1H-purine-2,6-dione;
3-cyclopropyl-3,7-dihydro-7-(2-oxopropyl)-1H-purine-2,6-dione;
3-cyclopropyl-3,7-dihydro-1-methyl-7-(2-oxopropyl)-1H-purine2,6-dione;
3-cyclopropyl-1-ethyl-3,7-dihydro-7-(2-oxopropyl)-1H-purine-2, 6-dione;
3-cyclopropyl-3,7-dihydro-7-(2-oxopropyl)-1-n-propyl-1H-purine-2,6-dione;
1-n-butyl-3-cyclopropyl-3,7-dihydro-7-(2-oxopropyl)-1H-purine-2-6-dione;
3-cyclopropyl-3,7-dihydro-7-(2-oxopropyl)-1-n-pentyl-1H-purine-2,6-dione;

3-cyclopropyl-1-n-hexyl-3,7-dihydro-7-(2-oxopropyl)-1H-purine-2,6-dione;
3-cyclopropyl-3,7-dihydro-7-(3-oxobutyl)-1H-purine-2,6-dione;
3-cyclopropyl-3,7-dihydro-1-methyl-7-(3-oxobutyl)-1H-purine-2,6-dione;
3-cyclopropyl-1-ethyl-3,7-dihydro-7-(3-oxobutyl)-1H-purine-2,6-dione;
3-cyclopropyl-3,7-dihydro-7-(4-oxopentyl)-1H-purine-2,6-dione;
3-cyclopropyl-3,7-dihydro-1-methyl-7-(4-oxopentyl)-1H-purine-2,6-dione;
3-cyclopropyl-3,7-dihydro-7-(5-oxohexyl)-1H-purine-2,6-dione;
3-cyclopropyl-3,7-dihydro-1-methyl-7-(5-oxohexyl)-1H-purine-2,6-dione;
3-cyclopropyl-1-ethyl-3,7-dihydro-7-(5-oxohexyl)-1H-purine-2,6-dione;
3-cyclopropyl-3,7-dihydro-7-(5-oxohexyl)-1-n-propyl-1H-purine2,6-dione;
1-n-butyl-3-cyclopropyl-3,7-dihydro-7-(5-oxohexyl)-1H-purine-2,6-dione;
3-cyclopropyl-3,7-dihydro-7-(5-oxohexyl)-1-n-pentyl-1H-purine-2,6-dione;
3-cyclopropyl-1-n-hexyl-3,7-dihydro-7-(5-oxohexyl)-1H-purine-2,6-dione;
3-cyclopropyl-3,7-dihydro-7-methyl-1-[(2-methyl-1,3-dioxolan-2-yl)methyl]-1H-purine-2,6-dione;
3-cyclopropyl-3,7-dihydro-7-methyl-1-[4-(2-methyl-1,3-dioxolan-2-yl)butyl]-1H-purine-2,6-dione;
3-cyclopropyl-3,7-dihydro-1-methyl-7-[(2-methyl-1,3-dioxolan-2yl)methyl]-1H-purine2,6-dione; and
1-n-butyl-3-cyclopropyl-3,7-dihydro-7-[(2-methyl-1,3-dioxolan-2-yl)methyl]-1H-purine-2,6-dione.

Examples of the pharmacologically acceptable salts of the compound of the present invention represented by the formula (I) and (II) may be inorganic salts such as, for example, sodium, potassium, calcium, or ammonium salts; or organic salts such as, for example, etylenediamine salt, ethanolamine salt, N,N-dialkylethanolamine salt, or triethanolamine salt.

According to the present invention, various methods for preparing the novel xanthine compound of the present invention represented by the above-descrived formula (I) and (II) are provided.

According to one embodiment of the process of the present invention, the xanthine compound of the present invention represented by formula (I) wherein $R^1$ represents 4-methoxybenzyl group, a straight- or branched chain alkyl group having 1 to 6 carbon atoms, or a cycloalkyl group having 3 to 6 carbon atoms, and A represents $-COCH_3$; and the xanthine compound of the present invention represented by formula (II) wherein A represents $-COCH_3$ can be prepared by reacting in an organic solvent a xanthine compound represented by the following formula (III) or (IV):

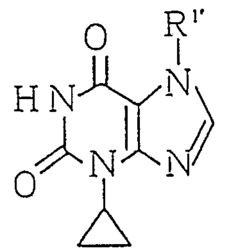

(III)

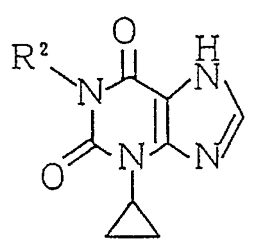

(IV)

wherein $R^1$ represents 4-methoxybenzyl group, a straight- or branched chain alkyl group having 1 to 6 carbon atoms, or a cycloalkyl group having 3 to 6 carbon atoms, and $R^2$ is the same as those defined above, with a halogenated alkyl compound represented by the following formula (V):

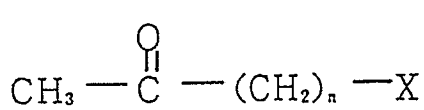

(V)

wherein n is the same as those defined above, and X represents a halogen atom, in the presence of a base

as dehydrohalogenating agent.

Examples of the organic solvent used in the above-descrived process include alcohols such as, for examle, methanol, ethanol, n-propanol, isopropanol, or n-butanol; aprotic polar solvents such as, for example, tetrahydrofuran, acetonitrile, N,N-dimethylformamide, N-methyl-2-pyrrolidone, and dimethyl sulfoxide. Examples of the base used in the above-descrived process include, for example, metallic sodium, sodium hydride, sodium amide, sodium hydroxide, potassium hydroxide, sodium carbonate, and potassium carbonate. The reaction may be carried out at a temperature of from an ice-cooled temperature to the reflux temperature of the reaction solvent used.

The starting material represented by the above formula (III) used in the above process may be prepared according to the following process:

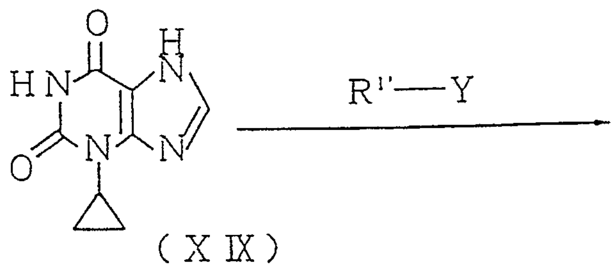


(III)

(XIX)

wherein $R^{1'}$ is the same as those defined above, and Y represents a halogen atom.

The compound represented by formula (XIX) is disclosed in the Japanese Patent Unexamined Publication No. 55-57589 and can be preprared according to the method described in the patent document.

The starting material represented by the above formula (IV) used in the above-described process wherein $R^2$ is the same as those difined above except being a hydrogen atom may be prepared by the following method:

(XIX)

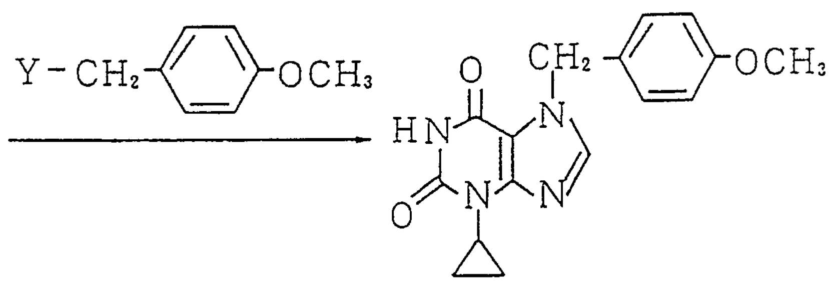

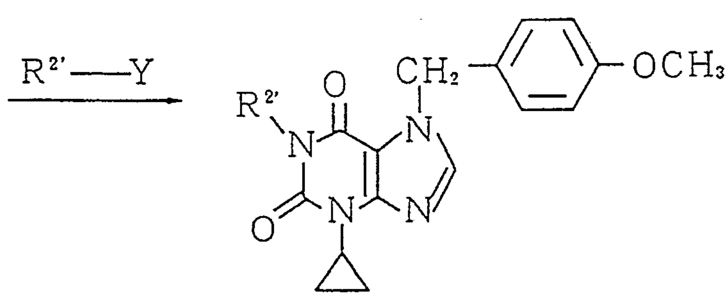


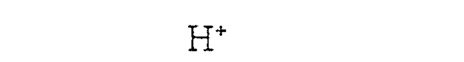


(IV)

wherein $R^{2'}$ represents a straight- or branched chain alkyl group having 1 to 6 carbon atoms or a cycloalkyl group having 3 to 6 carbon atoms, and Y is the same as those defined above.

According to another embodiment of the present invention, the compound represented by the above

formula (I) wherein $R^1$ represents 4-methoxybenzyl group, a straight- or branched-chain alkyl group having 1 to 6 carbon atoms, or a cycloalkyl group having 3 to 6 carbon atoms; n represent an integer of 2, and A represents $-COCH_3$; and the compound represented by the above formula (II) wherein n represent an integer of 2 and A represents $-COCH_3$ may be preprared by the Michael addition, i.e., the addition of methyl vinyl ketone to the xanthine compound represented by the above formula (III) or (IV) in the presence of a basic organic solvent.

The Michael reaction may be carried out according to the method well known to those skilled in the art at a temperature of from room temperature to the reflux temperature of the reaction solvent used by using the basic organic solvent such as, for example, pyridine, 2-picoline, or 2,6-lutidine.

According to yet another embodiment of the present invention, the compound represented by the above formula (I) wherein $R^1$ is represents a hydrogen atom and A represents $-COCH_3$ can be prepared by treating the compound represented by the above formula (I) wherein $R^1$ represents 4-methoxybenzyl group and A represents $-COCH_3$ with an acid in the presence of a scavenger in an organic solvent or without a solvent.

Examples of the organic solvent used in the above process of the present invention include, for example, benzene, toluene, xylene, carbon tetrachloride, and 1,2-dichloroethane; and examples of the acid scavenger include, for example, anisole and thioanisole. Examples of the acid include, for example, trifluoroacetic acid, trifluoromethanesulfonic acid, sulfuric acid, hydrochloric acid, and hydrobromic acid. The reaction may be carried out at a temperature of from room temperature to the reflux temperature of the reaction solvent used.

According to further embodiment of the present invention, the compound represented by the above formula (I) wherein $R^1$ represents 4-methoxybenzyl group, a straight- or branched-chain alkyl group having 1 to 6 carbon atoms, or a cycloalkyl group, having 3 to 6 carbon atoms, and A represents $-COCH_3$ ; and the compound represented by the above formula (II) wherein $R^2$ represents a straight- or branched-chain alkyl group having 1 to 6 carbon atoms, or a cycloalkyl group having 3 to 6 carbon atoms, and A represents $-COCH_3$ can be prepared by reacting in an organic solvent a xanthine compound represented by the following formula (XII) or (XIII):

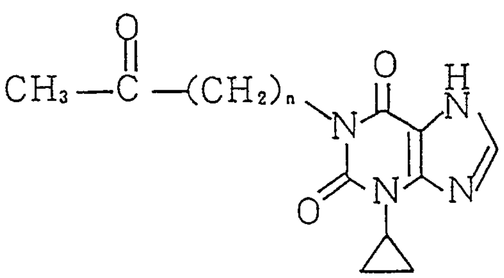

( X II )

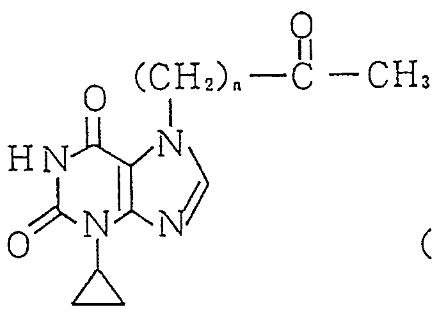

( X III )

wherein n is the same as those defined above, with a halogenated alkyl compound represented by the following formula (XIV):

$$R^{1'}—Y \quad \text{or} \quad R^{2'}—Y \qquad (XIV)$$

wherein $R^{1'}$, $R^{2'}$, and Y are the same as those defined above in the presence of a base as dehydrohalogenating agent.

Examples of the organic solvent used in the above process include alcohols such as, for example, methanol, ethanol, n-propanol, isopropanol, or n-butanol; and aprotic polar solvents such as, for example, tetrahydrofuran, acetonitrile, N,N-dimethylformamide, N-methyl-2-pyrrolidone, or dimethyl sulfoxide. Examples of the base used in the above-described process include, for example, metallic sodium, sodium hydride, sodium amide, sodium hydroxide, potassium hydroxide, sodium carbonate, and potassium carbonate. The reaction may be carried out at a temperature of from an ice-cooled temperature to the reflux temperature of the reaction solvent used.

According to still further embodiment of the present invention, the compound represented by the above formula (I) or (II) wherein A represents $-C(OR^3)(OR^4)CH_3$ can be prepared by converting the compound represented by the above formula (I) or (II) wherein A represents $-COCH_3$ to a corresponding ketal compound in the presence of an acid as a catalyst in an organic solvent.

The above process for preparing the ketal compound may be carried out according to the method well known to those skilled in the art. Examples of the organic solvent include, for example, benzene, toluene, or xylene; and exmaples of the acid as a catalyst include, for example, p-toluenesulfonic acid or pyridinium p-toluenesulfonate. The reaction may be carried out at about the reflux temperature of the solvent used in the process.

The novel xanthine compound of the present invention represented by the above formula (I) or (II) and a pharmacologically acceptable salts thereof has a bronchodilating activity and improving activity for brain disfunction, and thus is useful for, the treatment of, for example, respiratory tract disease or brain disfunction.

The xanthine compounds of the present invention and their pharmacologically acceptable salts may be administered orally or parenterally to a patient preferably as a pharmaceutical composition for the treatment of respiratory tract disease or brain disfunction comprising the effective amount of the xanthine compound together with a pharmaceutically acceptable carrier or coating.

The pharmaceutical composition may be in the form of, for example, capsule, tablet, substilized granule, granule, syrup, or powder suitable for oral administration; injection, suppository, ointment, or cataplasm suitable for parenteral administration. The pharmaceutical composition may be prepared according to the method well known to those skilled in the art by using pharmacologically and pharmaceutically acceptable carriers or coatings. For the preparation of the pharmaceutical composition of the present invention suitable for oral administration or suppository, the carrier or coating may comprise a diluent such as, for example, lactose, D-mannitol, starch, or crystalline cellulose; a disintegrant such as, for example, carboxymethylcellulose or calcium carboxymethylcellulose; a binder such as, for example, hydroxypropylcellulose, hydroxypropylmethylcellu lose, or polyvinylpyrrolidone; a lubricant such as, for example, magnesium stearate or talc; a coating agent such as, for example, hydroxypropylmethylcellulose, titanium oxide, or sucrose; or a base such as, for example, polyethyleneglycol or hard fat. The pharmaceutical composition of the present invention suitable for injection may comprise the following: a solubilizing agent or a solubilizer, e.g., distilled water for injection, saline, or propyleneglycol which is useful for an aqueous composition or a composition for preparing aqueous solution before use; an isotonicity agent such as, for example, glucose, sodium chloride, D-mannitol, or glycerin; pH adjusting agent such as, for example, an inorganic or organic acid or an inorganic or organic base; and a stabilizer. The pharmaceutical composition of the present invention suitable for dermal administration may comprise th following: a base such as, for example, petrolatum, liquid paraffin, or polyethyleneglycol; an adhesive such as, for example, sodium polyacrylate, polyvinylalcoholm ethylcellulose, or polybutene; and a base sheet such as, for example, cloth.

The dose of the pharmaceutical composition of the present invention for an adult patient may generally be from about 10 to 1,000 mg per day for oral administration, which may be increased or decreased depending on the conditions of the patient to be treated.

Action

As an example to show the excellent effect of the compounds of the present invention, the results of relaxation activity in the isolated guinea-pig trachea obtained by monitoring bronchodilative activity are summarrized in Table 1. The results of relaxation activity in the isolated dog mesenteric artery and protecting effect on complete cerebral ischemia in mice obtained by monitoring improved activity for brain disfunction are also summarized in Tables 2 and 3, respectively. In addition, the results of chronotropic activity in the isolated guinea-pig atria obtained by monitoring undesirable side-effects are summarized in Table 4. In the experiments, the following compounds were used as the reference compounds.

Reference compound A : theophylline

Reference compound B : propentofylline

1.Relaxation activity in the isolated guinea-pig trachea

Male Hartley guinea-pigs weighing about 500 g were sacrificed and the trachea were removed. The tracheal chain strips were made and the preparations were mounted vertically in an organ bath containing Locke-Ringer solution. The bath medium was maintained at 37° C and was equilibrated with a gas mixture consisting of 95% $O_2$ and 5% $CO_2$. Relaxation activities of the test compounds on the resting tonus of the preparations were recorded isotonically. The test compounds were added cumulatively to the bath. $ED_{50}$ values representing the amount of the compound tested which produces 50% relaxations of the maximal relaxations ($10^{-6}$M isoproterenol) were calculated on the basis of the results obtained above.

The results are shown in Table 1.

Table 1

| Test compound | relaxation activity in the isolated guinea-pig trachea $ED_{50}$ ( $\times 10^{-5}$ M) | relative effectiveness of the compounds to reference A |
|---|---|---|
| Example 15 | 1.8 | 1.6 |
| Example 16 | 2.3 | 1.2 |
| Example 17 | 1.1 | 2.5 |
| Example 26 | 1.4 | 2.0 |
| Example 30 | 0.15 | 19 |
| Example 34 | 0.60 | 4.7 |
| Example 40 | 1.6 | 1.8 |
| Reference compound A | 2.8 | 1 |

As shown in Table 1, the compounds of the present invention exhibited a more potent brochodilative activity than the reference compound A (theophylline).

2.Relaxation activity in the isolated dog mesenteric artery

The mesenteric arteries were isolated from mongrel dogs of either sex weighing about 10kg. Each helical strip (2 mm in width and 20 mm in length) was mounted vertically in an organ bath containing Krebs-Henseleit solution. The bath medium was maintained at 37 ° C and was equilibrated with a gas mixture consisting of 95% $O_2$ and 5% $CO_3$. A resting tension of 1.0 g was applied and the response was recorded isometrically. The preparations were pre-contracted with prostaglandin $F_2\alpha$ ($3 \times 10^{-6}$ M) and the smooth muscle relaxations induced by the test compounds were recorded. The test compounds were added cumulatively to the bath. $ED_{50}$ values representing the amount of the compound which produces 50% relaxations of the maximal relaxations ($10^{-4}$M papaverine) were caluculated on the basis of the results obtained above.

The results are shown in Table 2.

Table 2

| Test compound | relaxation activity in the isolated dog mesenteric artery $ED_{50}$ ( $\times 10^{-5}$ M) | relative effectiveness of the compounds to reference B |
|---|---|---|
| Example 6 | 1.5 | 2.5 |
| Example 15 | 3.6 | 1.1 |
| Example 17 | 1.8 | 2.1 |
| Example 26 | 1.0 | 3.8 |
| Example 30 | 0.056 | 68 |
| Example 34 | 0.064 | 59 |
| Reference compound B | 3.8 | 1 |

3.Protective effect on complete cerebral ischemia in mice

Male ddY mice weighing about 25 g were used. Complete cerebral ischemia was produced by the amputation of the cervix between the medulla oblongata and the spinal cord. The time between decapitation and the last gasp was recorded as gasping duration. Test compounds were suspended with 0.5% carboxymethylcellulose except propentofylline which was dissolved in distilled $H_2O$, and then administered orally 30 minutes before the decapitation. The gasping duration was evaluated at the dose of 50 mg/kg.

The results are shown in Table 3.

Table 3

| Test compound | protective effect on complete cerebral iscehemia in mice gasping duration(%, 50 mg/kg,p.o.) |
|---|---|
| Example 15 | 112.3 |
| Example 16 | 107.8 |
| Example 17 | 106.8 |
| Reference compound B | 106.4 |

As shown in Tables 2 and 3, the compounds of the present invention exhibited a more improved activity for brain disfunction than the reference compound B(propentofylline).

4.Chronotropic activity in the isolated guinea-pig atrium

Male Hartley guinea-pig weighing about 500 g was sacrificed and the right atrium was removed. The atrium was mounted vertically in an organ bath containing Krebs-Henseleit solution. The bath medium was maintained at 30$^{\circ}$C and was equilibrated with a gas mixture consisting of 95% $0_2$ and 5% $CO_2$. A restintion of 0.5 g was applied. The spontaneous beating rates for the right atrium were recorded by using a heart rate counter. The test compounds were added cumulatively to the bath. $ED_{15}$ values representing the amount of the compound which produced 15% increases of the spontaneous beating rates for the right atria were caluculated on the basis of the results obtained.

The results are shown in Table 4.

Table 4

| Test compound | chronotropic activity in the isolated guinea-pig atria $ED_{15}$ ($\times 10^{-5}$ M) |
|---|---|
| Example 3 | > 10 |
| Example 6 | > 10 |
| Example 15 | > 10 |
| Example 16 | > 10 |
| Example 17 | > 10 |
| Example 25 | > 10 |
| Example 30 | > 10 |
| Example 34 | > 10 |
| Example 38 | > 10 |
| Example 40 | > 10 |
| Reference compound A | 7.6 |
| Reference compound B | 5.1 |

As shown in Table 4, the compound of the present invention exhibited a less potent chronotropic

activity than the reference compounds A (theophylline) and B (propentofylline).

Examples

The present invention will be further illustrated by the following Examples and Reference Examples. The Examples are given by the way of illustration only and are not to be construed as limiting.

Reference 1
3-Cyclopropyl-3,7-dihydro-7-(4-methoxybenzyl)-1H-purine-2-6-dione

A mixture of 4.00 g of 3-cyclopropyl-3,7-dihydro-1H-purine-2, 6-dione, 3.10 ml of 4-methoxybenzyl chloride, and 2.87 g of potassium carbonate in 30 ml of N,N-dimethylformamide was stirred at 60 ° C for 2. 5 hours. The reaction mixture was poured into ice-cold water and the resulting mixture was adjusted to pH 5 with 10% hydrochloric acid. Precipitates formed were collected by filtration and washed with water and isopropyl ether, successively, to give 5.65 g of pale yellow crystals, which were recrystallized from N,N-dimethylformamide and ethanol to give colorless needles, mp 224.5 - 226 ° C.

| Analysis for $C_{16}H_{16}N_4O_3$: | | | |
|---|---|---|---|
| Calculated | C,61.53; | H,5.16; | N,17.94 |
| Found | C,61.48; | H,5.36; | N,17.85 |

The compounds of References 2 to 7 were prepared in the same manner as described in Reference 1.

Reference 2
3-cyclopropyl-3,7-dihydro-7-methyl-1H-purine-2,6-dione.

Pale brown needles, mp 288 - 290 ° C (DMF)

| Analysis for $C_9H_{10}N_4O_2$: | | | |
|---|---|---|---|
| Calculated | C,52.42; | H,4.89; | N,27.17 |
| Found | C,52.19; | H,4.98; | N,26.98 |

Reference 3
3-Cyclopropyl-7-ethyl-3,7-dihydro-1H-purine-2,6-dione

Colorless needles, mp 227.5 - 228.5 ° C (EtOH)

| Analysis for $C_{10}H_{12}N_4O_2$: | | | |
|---|---|---|---|
| Calculated | C,54.54; | H,5.49; | N,25.44 |
| Found | C,54.61; | H,5.60; | N,25.48 |

Reference 4
3-cyclopropyl-3,7-dihydro-7-n-propyl-1H-purine-2,6-dione

Colorless needles, mp 165 - 166 ° C (EtOH)

| Analysis for $C_{11}H_{14}N_4O_2$: | | | |
|---|---|---|---|
| Calculated | C,56.40; | H,6.02; | N,23.92 |
| Found | C,56.28; | H,6.07; | N,23.96 |

Reference 5
7-n-Butyl-3-cyclopropyl-3,7-dihydro-1H-purine-2,6-dione

Slightly yellow needles, mp 154 - 155.5 °C (EtOH)

| Analysis for $C_{12}H_{16}N_4O_2$: | | | |
|---|---|---|---|
| Calculated | C,58.05; | H,6.50; | N,22.57 |
| Found | C,57.81; | H,6.50; | N,22.37 |

Reference 6
3-Cyclopropyl-3,7-dihydro-7-n-pentyl-1H-purine-2,6-dione

Pale yellow prisms, mp 128 - 129.5° C (iso-PrOH)

| Analysis for $C_{13}H_{18}N_4O_2$: | | | |
|---|---|---|---|
| Calculated | C,59.53; | H,6.92; | N,21.36 |
| Found | C,59.60; | H,6.77; | N,21.33 |

Reference 7
3-Cyclopropyl-7-n-hexyl-3,7-dihydro-1H-purine-2,6-dione

Slightly yellow plates, mp 130.5 - 131.5° C (EtOH)

| Analysis for $C_{14}H_{20}N_4O_2$: | | | |
|---|---|---|---|
| Calculated | C,60.85; | H,7.29; | N,20.27 |
| Found | C,60.87; | H,7.24; | N,20.01 |

Reference 8
3-Cyclopropyl-1-ethyl-3,7-dihydro-1H-purine-2,6-dione
a)3-Cyclopropyl-1-ethyl-3,7-dihydro-7-(4-methoxybenzyl)-1H-purine-2,6-dione

A mixture of 5.00 g of 3-cyclopropyl-3,7-dihydro-7-(4-methoxybenzyl)-1H-purine-2,6-dione, 1.54 ml of ethyl iodide, and 2.65 g of potassium carbonate in 30 ml of N,N-dimethylformamide was stirred at 70 °C for 6 hours. The reaction mixture was poured into ice-cold water. Precipitates formed were collected by filtration, washed with water and n-hexane, successively, to give 5.00 g of pale yellow crystals, which were recrystallized from isopropanol to give colorless needles, mp 134.5 - 136 °C

| Analysis for $C_{18}H_{20}N_4O_3$: | | | |
|---|---|---|---|
| Calculated<br>Found | C,63.52;<br>C,63.57; | H,5.92;<br>H,5.84; | N,16.46<br>N,16.38 |

b)3-Cyclopropyl-1-ethyl-3,7-dihydro-1H-purine-2,6-dione

To a mixture of 4.50 g of 3-cyclopropyl-1-ethyl-3,7-dihydro-7-(4-methoxybenzyl)-1H-purine-2.6-dione and 2.16 ml of anisole in 22.5 ml of trifluoroacetic acid, 0.30 ml of concentrated sulfuric acid (98%) was added dropwise at room temperature with stirring, and then the mixture was refluxed for 12 hours. The reaction mixture was concentrated under the reduced pressure. Water and isopropyl ether was added to an oily residue, and the resulting mixture was adjusted to pH 5 with 20% aqueous sodium hydroxide solution. Precipitates formed were collected by filtration, and washed with water and isopropyl ether, successively, to give 2.12 g of pale brown crystals, which were recrystallized from ethanol to give colorless needles, mp 245 - 248° C.

| Analysis for $C_{10}H_{12}N_4O_2$: | | | |
|---|---|---|---|
| Calculated<br>Found | C,54.54;<br>C,54.59; | H,5.49;<br>H,5.80; | N,25.44<br>N,25.31 |

Example 1
3-Cyclopropyl-3,7-dihydro-7-(4-methoxybenzyl)-1-(5-oxohexyl)-1H-purine-2,6-dione

A mixture of 4.00 g of 3-cyclopropyl-3,7-dihydro-7-(4-methoxybenzyl)-1H-purine-2,6-dione, 2.75 g of 5-oxohexyl bromide and 2. 13 g of potassium carbonate in 26 ml of N,N-dimethylformamide was stirred at 60° C for 9.5 hours. The reaction mixture was concentrated under the reduced pressure, and water was added to the residue. Precipitates formed were collected by filtration and washed with water and n-hexane, successively, to give 5.00 g of colorless crystals, which were recrystallized from ethyl acetate-n-hexane to give colorless needles, mp 113 - 114.5° C.

| Analysis for $C_{22}H_{26}N_4O_4$: | | | |
|---|---|---|---|
| Calculated<br>Found | C,64.38;<br>C,64.24; | H,6.38;<br>H,6.26; | N,13.65<br>N,13.47 |

Example 2
3-Cyclopropyl-3,7-dihydro-1-(5-oxohexyl)-1H-purine-2,6-dione

To a mixture of 4.50 g of 3-cyclopropyl-3,7-dihydro-7-(4-methoxybenzyl)-1-(5-oxohexyl)-1H-purine-2,6-dione and 1.67 ml of anisole in 20 ml of trifluoroacetic acid, 0.20 ml of concentrated sulfuric acid (98%) was added dropwise at room temperature with stirring, and then the mixture was refluxed for 10 hours. The reaction mixture was concentrated under a reduced pressure. Water and isopropyl ether was added to an oily residue, and the resulting mixture was adjusted to pH 6 with 10% aqueous sodium hydroxide solution. Precipitates formed were collected by filtration and washed with water and n-hexane, successively, to give 2.55 g of slightly brown crystals, which were recrystallized from ethanol to give colorless crystals, mp 203 - 205 °C

| Analysis for $C_{14}H_{18}N_4O_3$: | | | |
|---|---|---|---|
| Calculated | C,57.92; | H,6.25; | N,19.30 |
| Found | C,57.97; | H,6.19; | N,19.19 |

Example 3
3-Cyclopropyl-3,7-dihydro-7-methyl-1-(2-oxopropyl)-1H-purine-2,6-dione

To a cooled mixture of 3.80 g of 3-cyclopropyl-3,7-dihydro7-methyl-1H-purine-2,6-dione and 3.06 g of potassium carbonate in 38 ml of N,N-dimethylformamide, 1.85 ml of bromoacetone was added dropwise under stirring, and then the mixture was stirred at room temperature for 3 days. The reaction mixture was concentrated under a reduced pressure, and then water was added to the residue. Precipitates formed were collected by filtration and washed with water and n-hexane, successively, to give 3.18 g of pale brown crystals, which were recrystallized with ethyl acetate to give pale brown prisms, mp 183 - 185 °C.

| Analysis for $C_{12}H_{14}N_4O_3$: | | | |
|---|---|---|---|
| Calculated | C,54.96; | H,5.38; | N,21.36 |
| Found | C,54.85; | H,5.66; | N,21.35 |

The compounds of Examples 4 to 17 were prepared in the same manner as described in Example 3.

Example 4
3-Cyclopropyl-3,7-dihydro-7-methyl-1-(3-oxobutyl)-1H-purine-2,6-dione

Colorless crystals, mp 145 - 147 °C (AcOEt)

| Analysis for $C_{13}H_{16}N_4O_3$: | | | |
|---|---|---|---|
| Calculated | C,56.51; | H,5.84; | N,20.28 |
| Found | C,56.43; | H,5.79; | N,20.31 |

Example 5
3-Cyclopropyl-3,7-dihydro-7-methyl-1-(4-oxopentyl)-1H-purine-2,6-dione

Pale brown crystals, mp: 86.5 - 88 °C (AcOEt/n-Hexane)

| Analysis for $C_{14}H_{18}N_4O_3$: | | | |
|---|---|---|---|
| Calculated | C,57.92; | H,6.25; | N,19.30 |
| Found | C,57.95; | H,6.29; | N,19.24 |

Example 6
3-Cyclopropyl-3,7-dihydro-7-methyl-1-(5-oxohexyl)-1H-purine-2,6-dione

Colorless columns, mp: 89 - 90 °C(iso-PrOH)

| Analysis for $C_{15}H_{20}N_4O_3$: | | | |
|---|---|---|---|
| Calculated | C,59.20; | H,6.62; | N,18.41 |
| Found | C,59.16; | H,6.66; | N,18.46 |

Example 7

3-Cyclopropyl-7-ethyl-3,7-dihydro-1-(2-oxopropyl)-1H-purine-2,6-dione

Brown columns, mp 163 - 165 °C (iso-PrOH/iso-$Pr_2O$)

| Analysis for $C_{13}H_{16}N_4O_3$: | | | |
|---|---|---|---|
| Calculated | C,56.51; | H,5.84; | N,20.28 |
| Found | C,56.27; | H,5.83; | N,20.23 |

Example 8

3-Cyclopropyl-7-ethyl-3,7-dihydro-1-(3-oxobutyl)-1H-purine-2,6-dione

Pale brown crystals, mp 130 - 132 °C(AcOEt)

| Analysis for $C_{14}H_{18}N_4O_3$: | | | |
|---|---|---|---|
| Calculated | C,57.92; | H,6.25; | N,19.30 |
| Found | C,57.85; | H,6.22; | N,19.29 |

Example 9

3-Cyclopropyl-7-ethyl-3,7-dihydro-1-(4-oxopentyl)-1H-purine-2,6-dione

Pale yellow needles, mp 91 - 93 °C (iso-$Pr_2O$)

| Analysis for $C_{15}H_{20}N_4O_3$: | | | |
|---|---|---|---|
| Calculated | C,59.20; | H,6.62; | N,18.41 |
| Found | C,59.03; | H,6.47; | N,18.20 |

Example 10

3-Cyclopropyl-7-ethyl-3,7-dihydro-1-(5-oxohexyl)-1H-purine-2,6-dione

Colorless needles, mp 98 - 101 °C (iso-PrOH)

| Analysis for $C_{16}H_{22}N_4O_3$: | | | |
|---|---|---|---|
| Calculated | C,60.36; | H,6.96; | N,17.60 |
| Found | C,60.19; | H,6.77; | N,17.60 |

Example 11

3-Cyclopropyl-3,7-dihydro-1-(2-oxopropyl)-7-n-propyl-1H-purine-2,6-dione

Pale yellow plates, mp 123 - 125 °C (AcOEt/iso-$Pr_2O$)

| Analysis for $C_{14}H_{18}N_4O_3$: | | | |
|---|---|---|---|
| Calculated | C,57.92; | H,6.25; | N,19.30 |
| Found | C,57.95; | H,6.13; | N,19.58 |

Example 12
3-Cyclopropyl-3,7-dihydro-1-(3-oxobutyl)-7-n-propyl-1H-purine-2,6-dione

Colorless needles, mp 83 - 85 °C(iso-$Pr_2O$)

| Analysis for $C_{15}H_{20}N_4O_3$: | | | |
|---|---|---|---|
| Calculated | C,59.20; | H,6.62; | N,18.41 |
| Found | C,59.28; | H,6.61; | N,18.52 |

Example 13
3-Cyclopropyl-3,7-dihydro-1-(4-oxopentyl)-7-n-propyl-1H-purine-2,6-dione

Colorless plates, mp 65 - 66 °C (iso-$Pr_2O$)

| Analysis for $C_{16}H_{22}N_4O_3$: | | | |
|---|---|---|---|
| Calculated | C,60.36; | H,6.96; | N,17.60 |
| Found | C,60.28; | H,6.96; | N,17.48 |

Example 14
3-Cyclopropyl-3,7-dihydro-1-(5-oxohexyl)-7-n-propyl-1H-purine-2,6-dione

Colorless crystals, mp 84 - 86 °C (iso-PrOH)

| Analysis for $C_{17}H_{24}N_4O_3$: | | | |
|---|---|---|---|
| Calculated | C,61.43; | H,7.28; | N,16.86 |
| Found | C,61.26; | H,7.22; | N,16.74 |

Example 15
7-n-Butyl-3-cyclopropyl-3,7-dihydro-1-(5-oxohexyl)-1H-purine-2,6-dione

Colorless needles, mp 54 - 56 °C (iso-PrOH/iso-$Pr_2O$)

| Analysis for $C_{18}H_{26}N_4O_3$: | | | |
|---|---|---|---|
| Calculated<br>Found | C,62.41;<br>C,62.32; | H,7.56;<br>H,7.54; | N,16.17<br>N,16.08 |

Example 16
3-Cyclopropyl-3,7-dihydro-1-(5-oxohexyl)-7-n-pentyl-1H-purine-2,6-dione

Colorless needles, mp 51 - 53 °C (iso-PrOH/iso-$Pr_2O$)

| Analysis for $C_{19}H_{28}N_4O_3$: | | | |
|---|---|---|---|
| Calculated<br>Found | C,63.31;<br>C,63.30; | H,7.83;<br>H,7.88; | N,15.54<br>N,15.29 |

Example 17
3-Cyclopropyl-7-n-hexyl-3,7-dihydro-1-(5-oxohexyl)-1H-purine-2,6-dione

Colorless needles, mp: 49 - 51 °C (iso-$Pr_2O$)

| Analysis for $C_{20}H_{30}N_4O_3$: | | | |
|---|---|---|---|
| Calculated<br>Found | C,64.15;<br>C,64.21; | H,8.07;<br>H,8.15; | N,14.96<br>N,14.63 |

Example 18
3-Cyclopropyl-3,7-dihydro-7-(2-oxopropyl)-1H-purine-2,6-dione

To a mixture of 15.0 g of 3-cyclopropyl-3,7-dihydro-1-H- purine-2,6-dione and 13.0 g of potassium carbonate in 150 ml of N,N-dimethylformamide, 8.53 ml of bromoacetone was added dropwise at room temperature with stirring, and then the mixture was stirred at room temperature for 2.5 hours. The reaction mixture was poured into water, and then neutralized with 10% aqueous hydrochloric acid solution. Precipitates formed were collected by filtration and washed with water and diethyl ether, successively, to give 14.6 g of pale yellow crystals, which were recrystallized from N,N-dimethylformamide to give pale yellow needles, mp 277 - 278 °C.

| Analysis for $C_{11}H_{12}N_4O_3$: | | | |
|---|---|---|---|
| Calculated<br>Found | C,53.22;<br>C,53.30; | H,4.87;<br>H,4.92; | N,22.57<br>N,22.83 |

The compounds of Examples 19 to 21 were prepared in the same manner as described in Example 18.

Example 19
3-Cyclopropyl-3,7-dihydro-7-(3-oxobutyl)-1H-purine-2,6-dione

Pale brown needles, mp 181 - 183 °C (iso-PrOH)

| Analysis for $C_{12}H_{14}N_4O_3$: | | | |
|---|---|---|---|
| Calculated | C,54.96; | H,5.38; | N,21.36 |
| Found | C,54.87; | H,5.35; | N,21.36 |

Example 20
3-Cyclopropyl-3,7-dihydro-7-(4-oxopentyl)-1H-purine-2,6-dione

Pale yellow needles, mp 157.5 - 159 °C (MeOH)

| Analysis for $C_{13}H_{16}N_4O_3$: | | | |
|---|---|---|---|
| Calculated | C,56.51; | H,5.84; | N,20.28 |
| Found | C,56.27; | H,5.93; | N,20.41 |

Example 21
3-Cyclopropyl-3,7-dihydro-7-(5-oxohexyl)-1H-purine-2,6-dione

Pale yellow plates, mp 123 - 124.5 °C (iso-PrOH)

| Analysis for $C_{14}H_{18}N_4O_3$: | | | |
|---|---|---|---|
| Calculated | C,57.92; | H,6.25; | N,19.30 |
| Found | C,58.00; | H,6.34; | N,19.27 |

Example 22
3-Cyclopropyl-3,7-dihydro-1-methyl-7-(2-oxopropyl)-1H-purine-2,6-dione

To a cooled mixture of 2.40 g of 3-cyclopropyl-3,7-dihydro-7-(2-oxopropyl)-1H-purine-2,6-dione and 1.61 g of potassium carbonate in 24 ml of N,N-dimethylformamide, 0.72 ml of methyl iodide was added dropwise with stirring, and then the mixture was stirred at 60 °C for 1.5 hours. The reaction mixture was concentrated under a reduced pressure, and the residue was diluted with water. An organic product was extracted with ethyl acetate, and the organic layer was washed with water, dried over anhydrous sodium sulfate, and evaporated to give a solid product. The solid product was collected and washed with n-hexane to give 2.29 g of pale brown crystals, which was recrystallized from MeOH to give slightly brown prisms, mp 172 - 173.5 °C.

| Analysis for $C_{12}H_{14}N_4O_3$: | | | |
|---|---|---|---|
| Calculated | C,54.96; | H,5.38; | N,21.36 |
| Found | C,54.92; | H,5.37; | N,21.57 |

The compounds of Examples 23 to 35 were prepared in the same manner as described in Example 22.

Example 23
3-Cyclopropyl-3,7-dihydro-1-methyl-7-(3-oxobutyl)-1H-purine-2,6-dione

Pale brown needles, mp 190.5 - 192 °C (iso-PrOH)

| Analysis for $C_{13}H_{16}N_4O_3$: | | | |
|---|---|---|---|
| Calculated<br>Found | C,56.51;<br>C,56.41; | H,5.84;<br>H,5.85; | N,20.28<br>N,20.24 |

Example 24
3-Cyclopropyl-3,7-dihydro-1-methyl-7-(4-oxopentyl)-1H-purine-2,6-dione

Pale yellow prisms, mp 97 - 98° C (iso-PrOH)

| Analysis for $C_{14}H_{18}N_4O_3$: | | | |
|---|---|---|---|
| Calculated<br>Found | C,57.92;<br>C,57.83; | H,6.25;<br>H,6.33; | N,19.30<br>N,19.29 |

Example 25
3-Cyclopropyl-3,7-dihydro-1-methyl-7-(5-oxohexyl)-1H-purine-2,6-dione

Colorless needles, mp 117 - 119 ° C (AcOEt)

| Analysis for $C_{15}H_{20}N_4O_3$: | | | |
|---|---|---|---|
| Calculated<br>Found | C,59.20;<br>C,59.17; | H,6.62;<br>H,6.69; | N,18.41<br>N,18.60 |

Example 26
3-Cyclopropyl-1-ethyl-3,7-dihydro-7-(2-oxopropyl)-1H-purine-2,6-dione

Colorless needles, mp 202 - 202.5 ° C(MeOH)

| Analysis for $C_{13}H_{16}N_4O_3$: | | | |
|---|---|---|---|
| Calculated<br>Found | C,56.51;<br>C,56.31; | H,5.84;<br>H,5.82; | N,20.28<br>N,20.30 |

Example 27
3-Cyclopropyl-1-ethyl-3,7-dihydro-7-(5-oxohexyl)-1H-purine-2,6-dione

Slightly yellow plates, mp 87 - 88.5° C (AcOEt/n-hexane)

| Analysis for $C_{16}H_{22}N_4O_3$: | | | |
|---|---|---|---|
| Calculated<br>Found | C,60.36;<br>C,60.28; | H,6.96;<br>H,6.88; | N,17.60<br>N,17.56 |

Example 28

3-Cyclopropyl-3,7-dihydro-7-(2-oxopropyl)-1-n-propyl-1H-purine-2,6-dione

Colorless needles, mp 198.5 - 199.5 °C (MeOH)

| Analysis for $C_{14}H_{18}N_4O_3$: | | | |
|---|---|---|---|
| Calculated | C,57.92; | H,6.25; | N,19.30 |
| Found | C,57.71; | H,6.15; | N,19.34 |

Example 29
3-Cyclopropyl-3,7-dihydro-7-(5-oxohexyl)-1-n-propyl-1H-purine-2,6-dione

Colorless needles, mp 78 - 79.5 °C (AcOEt/n-Hexane)

| Analysis for $C_{17}H_{24}N_4O_3$: | | | |
|---|---|---|---|
| Calculated | C,61.43; | H,7.28; | N,16.86 |
| Found | C,61.43; | H,7.32; | N,16.86 |

Example 30
1-n-Butyl-3-cyclopropyl-3,7-dihydro-7-(2-oxopropyl)-1H-purine-2,6-dione

Colorless needles, mp 185.5 - 187 °C (MeOH)

| Analysis for $C_{15}H_{20}N_4O_3$: | | | |
|---|---|---|---|
| Calculated | C,59.20; | H,6.62; | N,18.41 |
| Found | C,58.95; | H,6.54; | N,18.12 |

Example 31
1-n-Butyl-3-cyclopropyl-3,7-dihydro-7-(5-oxohexyl)-1H-purine-2,6-dione

Colorless needles, mp 72 - 73.5 °C(AcOEt/n-Hexane)

| Analysis for $C_{18}H_{26}N_4O_3$: | | | |
|---|---|---|---|
| Calculated | C,62.41; | H,7.56; | N,16.17 |
| Found | C,62.26; | H,7.61; | N,16.39 |

Example 32
3-Cyclopropyl-3,7-dihydro-7-(2-oxopropyl)-1-n-pentyl-1H-purine-2,6-dione

Colorless needles, mp 131 - 132 °C (MeOH)

| Analysis for $C_{16}H_{22}N_4O_3$: | | | |
|---|---|---|---|
| Calculated | C,60.36; | H,6.96; | N,17.60 |
| Found | C,60.34; | H,6.94; | N,17.65 |

Example 33
3-Cyclopropyl-3,7-dihydro-7-(5-oxohexyl)-1-n-pentyl-1H-purine-2,6-dione

Colorless needles, mp 68 - 69 °C(AcOEt/n-Hexane)

| Analysis for $C_{19}H_{28}N_4O_3$: | | | |
|---|---|---|---|
| Calculated | C,63.31; | H,7.83; | N,15.54 |
| Found | C,63.26; | H,7.77; | N,15.49 |

Example 34
3-Cyclopropyl-1-n-hexyl-3,7-dihydro-7-(2-oxopropyl)-1H-purine-2,6-dione

Colorless needles, mp 134 - 136 °C (MeOH)

| Analysis for $C_{17}H_{24}N_4O_3$: | | | |
|---|---|---|---|
| Calculated | C,61.43; | H,7.28; | N,16.86 |
| Found | C,61.42; | H,7.32; | N,16.95 |

Example 35
3-Cyclopropyl-1-n-hexyl-3,7-dihydro-7-(3-oxohexyl)-1H-purine-2,6-dione

Pale brown needles, mp 67.5 - 69 °C (AcOEt/n-Hexane)

| Analysis for $C_{20}H_{30}N_4O_3$: | | | |
|---|---|---|---|
| Calculated | C,64.15; | H,8.07; | N,14.96 |
| Found | C,63.96; | H,8.11; | N,14.93 |

Example 36
3-Cyclopropyl-1-ethyl-3,7-dihydro-7-(3-oxobutyl)-1H-purine-2,6-dione

To a mixture of 1.33 g of 3-cyclopropyl-1-ethyl-3,7-dihydro-1H-purine-2,6-dione and 1.25 g of potassium carbonate in 14 ml of N,N-dimethylformamide, 0.96 g of 3-oxobutyl chloride was added dropwise at room temperature with stirring, and then the mixture was stirred at room temperature for one day. The reaction mixture was diluted with water and an organic product was extracted with dichloromethane. The organic layer was dried over anhydrous magnesium sulfate and evaporated to give a solid product. The solid product was collected and washed with n-hexane to give brown crystals, which were recrystallized from AcOEt/n-Hexane to give 1.20 g of pale brown crystals, mp 97 - 100 °C.

| Analysis for $C_{14}H_{18}N_4O_3$: | | | |
|---|---|---|---|
| Calculated | C,57.92; | H,6.25; | N,19.30 |
| Found | C,57.79; | H,6.25; | N,18.97 |

Example 37
3-Cyclopropyl-3,7-dihydro-7-methyl-1-[(2-methyl-1,3-dioxolan-2-yl)methyl]-1H-purine-2,6-dione

A mixture of 1.60 g of 3-cyclopropyl-3,7dihydro-7-methyl-1-(2-oxopropyl)-1H-purine-2,6-dione, 3.40 ml of ethylene glycol, and 1.16 g of p-toluenesulfonic acid monohydrate in 160 ml of benzene was refluxed for 43.5 hours using Dean-Stark apparatus. The reaction mixture was washed with aqueous sodium hydrogen carbonate solution, water, and saturated brine, succesively. The organic layer was dried over anhydrous magnesium sulfate and then evaporated to give 1.86 g of colorless needles, which were recrystallized from ethyl acetate to give colorless prisms, mp 130 - 135° C

| Analysis for $C_{14}H_{18}N_4O_4$: | | | |
|---|---|---|---|
| Calculated | C,54.89; | H,5.92; | N,18.29 |
| Found | C,54.97; | H,5.88; | N,18.59 |

The compounds of Examples 38 to 40 were prepared in the same manner as described in Example 37.

Example 38
3-Cyclopropyl-3,7-dihydro7-methyl-1-[4-(2-methyl-1,3-dioxolan-2-yl)butyl]-1H-purine-2,6-dione

Slightly yellow plates, mp 134 - 135.5° C (AcOEt)

| Analysis for $C_{17}H_{24}N_4O_4$: | | | |
|---|---|---|---|
| Calculated | C,58.61; | H,6.94; | N,16.08 |
| Found | C,58.51; | H,6.95; | N,15.93 |

Example 39
3-Cyclopropyl-3,7-dihydro-1-methyl-7-[(2-methyl-1,3-dioxolan-2-yl)methyl]-1H-purine-2,6-dione

Colorless prisms, mp 157 - 160° C (AcOEt)

| Analysis for $C_{14}H_{18}N_4O_4$: | | | |
|---|---|---|---|
| Calculated | C,54.89; | H,5.92; | N,18.29 |
| Found | C,54.87; | H,6.01; | N,18.27 |

Example 40
1-n-Butyl-3-cyclopropyl-3,7-dihydro-7-[(2-methyl-1,3-dioxolan-2-yl) methyl]-1H-purine-2,6-dione

Colorless needles, mp 69 - 71 ° C (AcOEt/n-Hexane)

| Analysis for $C_{17}H_{24}N_4O_4$: | | | |
|---|---|---|---|
| Calculated | C,58.61; | H,6.94; | N,16.08 |
| Found | C,58.50; | H,6.82; | N,16.07 |

Example 41

Tablets of a pharmaceutical preparation according to the present invention are prepared in the usual manner using the following constituents:

| | |
|---|---|
| Compound of the present invention | 50 mg |
| Lactose | q.s. |
| Corn starch | 15 mg |
| Magnesium stearate | 2 mg |
| Hydroxypropylmethylcellulose | 4.5 mg |
| Polyethyleneglycol 6000 | 0.3 mg |
| Titanium oxide | 0.2 mg |
| | 150 mg |

Example 42

Capsules of a pharmaceutical preparation according to the present invention are prepared in the usual manner by mixing the following constituents and filling in capsules:

| | |
|---|---|
| Compound of the present invention | 25 mg |
| Lactose | q.s. |
| Calcium carboxymethylcellulose | 15 mg |
| Hydroxypropylcellulose | 2 mg |
| Magnesium stearate | 1 mg |
| | 100 mg |

Example 43

Powders of a pharmaceutical preparation according to the present invention are prepared in the usual manner using the following constituents:

| | |
|---|---|
| Compound of the present invention | 100 mg |
| Lactose | q.s. |
| D-Mannitol | 500 mg |
| Hydroxypropylcellulose | 5 mg |
| Talc | 2 mg |
| | 1000 mg |

Example 44

Injections of a pharmaceutical preparation according to the present invention are prepared in the usual manner using the following constituents:

| | |
|---|---|
| Compounds of the present invention | 10 mg |
| Glucose | 1000 mg |
| Hydrochloric acid | q.s. |
| Distilled water for injection | q.s. |
| | 20 ml |

Example 45

Suppositories of a pharmaceutical preparation according to the present invention are prepared in the usual manner using the following constituents:

| | |
|---|---|
| Compounds of the present invention | 50 mg |
| Hard fat | 1150 mg |
| | 1200 mg |

Example 46

Ointment of a pharmaceutical preparation according to the present invention are prepared in the usual manner using the following constituents:

| | |
|---|---|
| Compounds of the present invention | 100 mg |
| Polyethyleneglycol 4000 | 500 mg |
| Polyethyleneglycol 400 | 400 mg |
| | 1000 mg |

## Claims

1. Xanthine compound represented by the following formula (I) or (II):

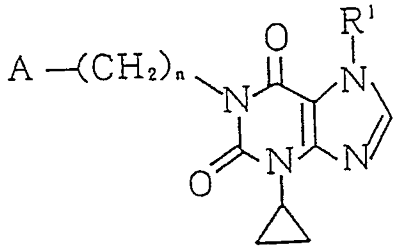

(I)

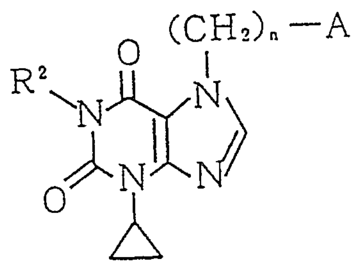

(II)

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms, and a pharmacologically acceptable salt of the above compound.

2. 3-cyclopropyl-3,7-dihydro-1-(5-oxohexyl)-1H-purine-2,6-dione and a pharmacologically acceptable salt thereof.

3. 3-cyclopropyl-3,7-dihydro-7-methyl-1-(5-oxohexyl)-1H-purine-2,6-dione and a pharmacologically acceptable salt thereof.

4. 3-Cyclopropyl-7-ethyl-3,7-dihydrol-(5-oxohexyl)-1H-purine-2,6-dione and a pharmacologically acceptable salt thereof.

5. 3-Cyclopropyl-3,7-dihydro-1-(5-oxohexyl)-7-n-propyl-1H-purine-2,6-dione and a pharmacologically acceptable salt thereof.

6. 7-n-Butyl-3-cyclopropyl-3,7-dihydro-1-(5-oxohexyl)-1H-purine-2,6-dione and a pharmacologically acceptable salt thereof.

7. 3-Cyclopropyl-3,7-dihydro-1-(5-oxohexyl)-7-n-pentyl-1H-purine-2,6-dione and a pharmacologically acceptable salt thereof.

8. 3-Cyclopropyl-7-n-hexyl-3,7-dihydro-1-(5-oxohexyl)-1H-purine-2,6-dione and a pharmacologically acceptable salt thereof.

9. 3-Cyclopropyl-3,7-dihydro-7-(2-oxopropyl)-1H-purine-2-6-dione and a pharmacologically acceptable salt thereof.

10. 3-Cyclopropyl-3,7-dihydro-1-methyl-7-(2-oxopropyl)-1H-purine-2,6-dione and a pharmacologically acceptable salt thereof.

11. 3-Cyclopropyl-1-ethyl-3,7-dihydro-7-(2-oxopropyl)-1H-purine-2,6-dione and a pharmacologically-acceptable salt thereof.

12. 3-Cyclopropyl-3,7-dihydro-7-(2-oxopropyl)-1-n-propyl-1H-purine-2,6-dione and a pharmacologically acceptable salt thereof.

13. 1-n-Butyl-3-cyclopropyl-3,7-dihydro-7-(2-oxopropyl)-1H-purine-2,6-dione and a pharmacologically acceptable salt thereof.

14. 3-Cyclopropyl-3,7-dihydro-7-(2-oxopropyl)-1-n-pentyl-1H-purine-2,6-dione and a pharmacologically acceptable salt thereof.

15. 3-Cyclopropyl-1-n-hexyl-3,7-dihydro-7-(2-oxopropyl)-1H-purine-2,6-dione and a pharmacologically acceptable salt thereof.

16. A process for preparing a xanthine compound represented by the following formula (VI) or (VII):

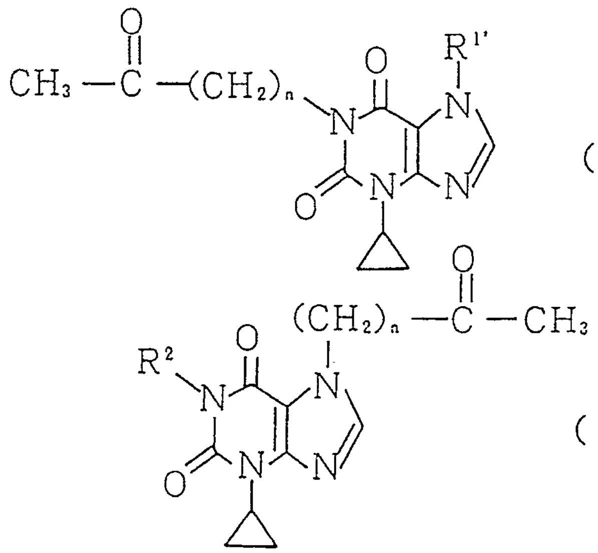

(VI)

(VII)

wherein $R^{1'}$ represents 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; and n represents an integer of from 1 to 4, or a pharmacologically acceptable salt thereof, comprising the step of reacting in an organic solvent a xanthine compound represented by the following formula (III) or (IV):

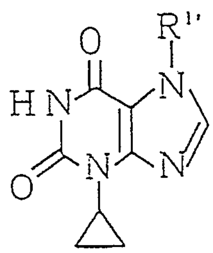

(III)

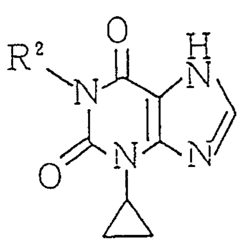

(IV)

wherein $R^{1'}$ and $R^2$ are the same as those defined above, with a halogenated alkyl compound represented by the following formula (V):

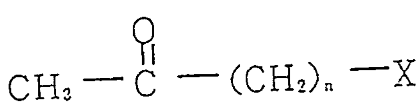

(V)

wherein n is the same as those defined above; and X represents a halogen atom, in the presence of a base as dehydrohalogenating agent.

17. A process for preparing a xanthine compound represented by the following formula (VIII) or (IX):

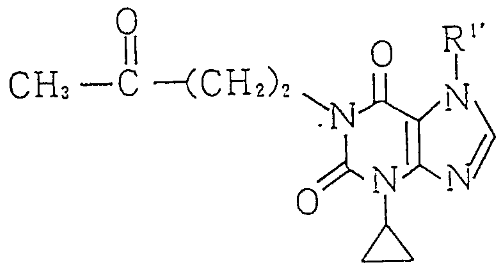

( VIII )

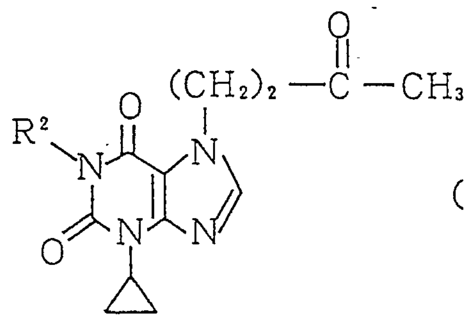

( IX )

wherein $R^{1'}$ represents 4-methoxybenzyl group, a straight- or branched-chain alkyl group having 1 to 6 carbon atoms, or a cycloalkyl group having 3 to 6 carbon atoms; and $R^2$ represents a hydrogen atom, a straight- or branched-chain alkyl group having 1 to 6 carbon atoms, or a cycloalkyl group having 3 to 6 carbon atoms, or a pharmacologically acceptable salt thereof, comprising the step of reacting a xanthine compound represented by the following formula (III) or (IV):

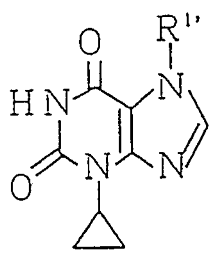

( III )

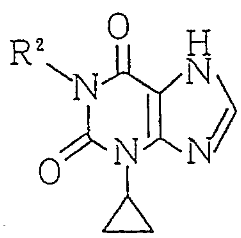

( IV )

wherein $R^{1'}$ and $R^2$ are the same as those defined above, with methyl vinyl ketone in a basic organic solvent.

18. A process for preparing a xanthine compound represented by the following formula (XI):

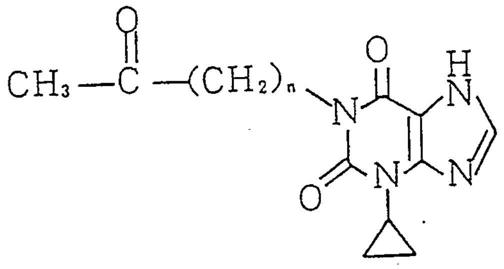
( X I )

wherein n represents an integer of from 1 to 4, or a pharmacologically acceptable salt thereof, comprising the step of treating a xanthine compound represented by the following formula (X):

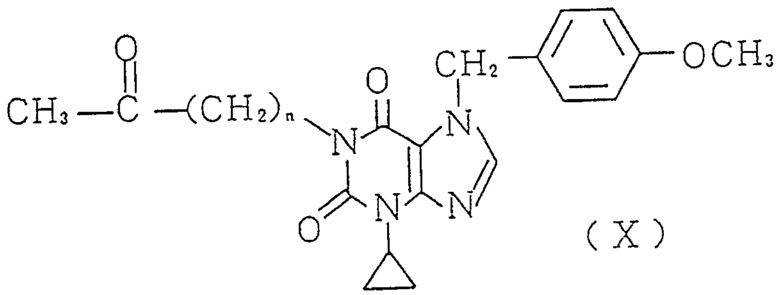
( X )

wherein n is the same as those defined above, with an acid in the presence of a scavenger in an organic solvent or without a solvent.

19. A process for preparing a xanthine compound represented by the following formula (VI) or (XV):

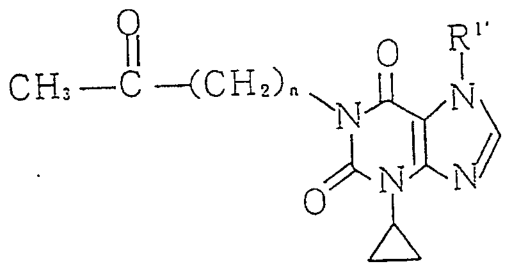
( VI )

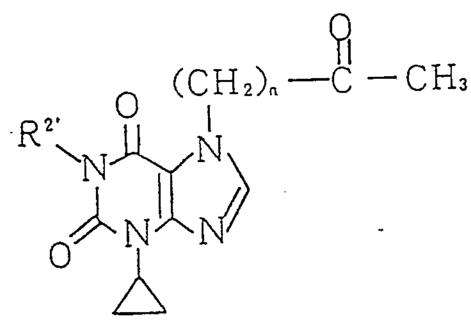
( X V )

wherein $R^{1'}$ represents 4-methoxybenzyl group, a straight- or branched-chain alkyl group having 1 to 6 carbon atoms, or a cycloalkyl group having 3 to 6 carbon atoms; $R^{2'}$ represents a straight- or branched-chain alkyl group having 1 to 6 carbon atoms or a cycloalkyl group having 3 to 6 carbon atoms; and n represents an integar of from 1 to 4, or a pharmacologically acceptable salt thereof, comprising the step of reacting a xanthine compound represented by the following formula (XII) or (XIII):

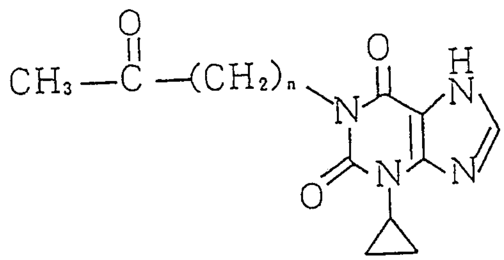

(XII)

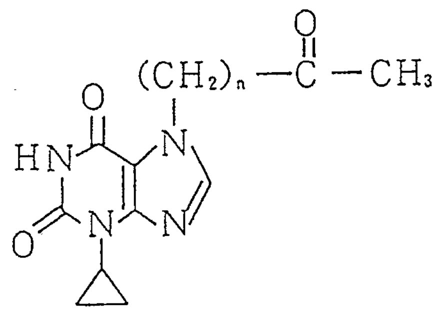

(XIII)

wherein n is the same as those defined above, with a halogenated alkyl compound represented by the following forumula (XIV):

$$R^{1'}—Y \quad \text{or} \quad R^{2'}—Y \qquad (XIV)$$

wherein $R^{1'}$ and $R^{2'}$ are the same as those defined above and Y represents a halogen atom, in an organic solvent in the presence of a base as a dehydrohalogenating agent.

20. A process for preparing a xanthine compound represented by the following formula (XVII) or (XVIII):

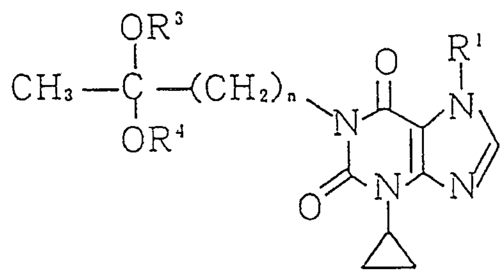

(XVII)

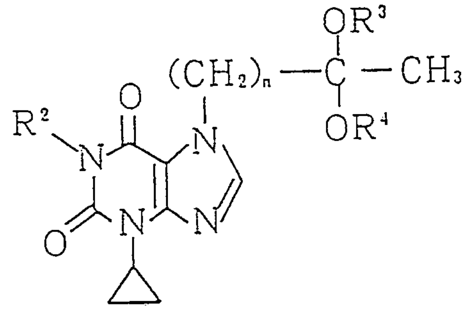

(XVIII)

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen

atom, a straight- or branched-chain alkyl group having 1 to 6 carbon atoms, or a cycloalkyl group having 3 to 6 carbon atoms; n represents an integer of from 1 to 4; $R^3$ and $R^4$ independently represent a alkyl group having 1 to 3 carbon atoms or $R^3$ together with $R^4$ represents a polymethylene group having 2 to 4 carbon atoms, or a pharmacologically acceptable salt thereof, comprising the step of converting a xanthine compound represented by the following formula (XVI) or (VII):

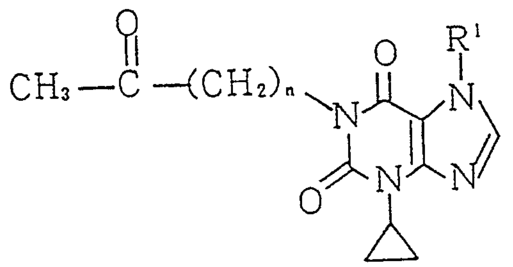

(XVI)

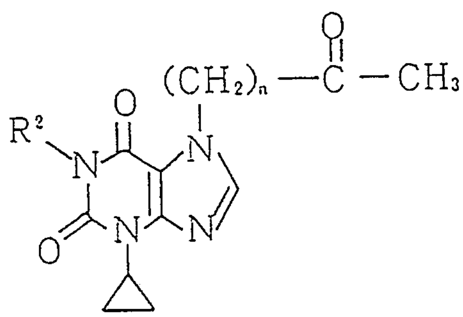

(VII)

wherein $R^1$, $R^2$, and n are the same as those defined above, to a corresponding ketal compound of (XVII) or (XVIII) in an organic solvent in the presence of an acid.

21. A bronchodilating agent comprising a xanthine compound represented by the following formula (I) or (II):

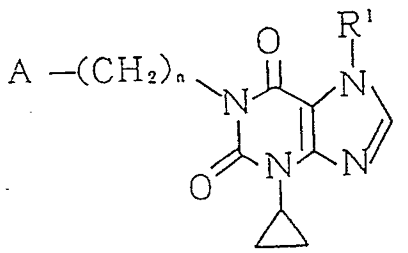

(I)

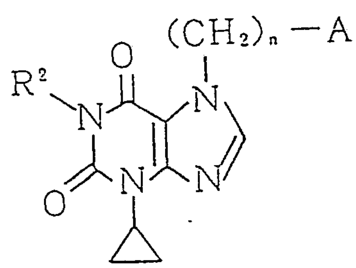

(II)

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms, and a pharmacologically acceptable salt of the above compound.

22. A brain function improving agent comprising a xanthine compound represented by the following formula

(I) or (II):

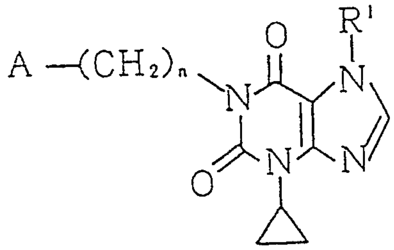

( I )

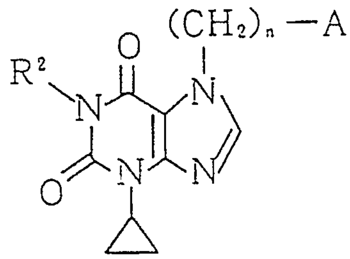

( II )

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms, and a pharmacologically acceptable salt of the above compound.

23. A pharmaceutical composition comprising an effective amount of a xanthine compound represented by the following formula (I) or (II):

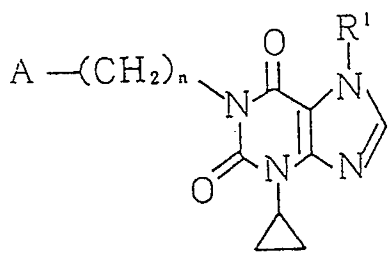

( I )

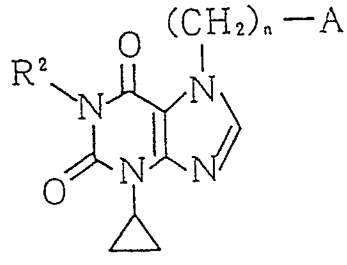

( II )

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms, or a pharmacologically acceptable salt of the above compound together with a pharmaceutically acceptable carrier or coating.

24. A method for the treatment of respiratory tract disease or brain disfunction comprising the step of administering to a mammal an effective amount of a substance selected from the group consisting essentially of (a) a xanthine compound represented by the following formula (I) or (II):

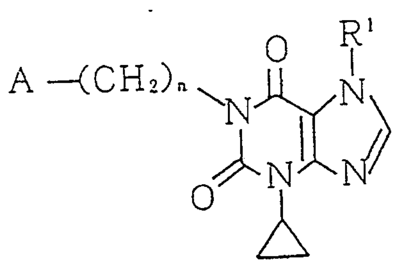

( I )

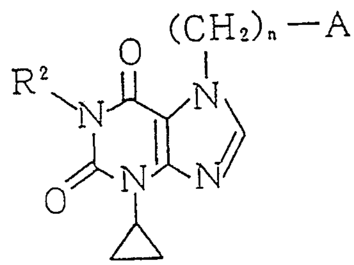

( II )

wherein $R^1$ represents a hydrogen atom, 4-methoxybenzyl group, a straight- or branched-chain alkyl group having 1 to 6 carbon atoms, or a cycloalkyl group having 3 to 6 carbon atoms; $R^2$ represents a hydrogen atom, a straight- or branched-chain alkyl group having 1 to 6 carbon atoms, or a cycloalkyl group having 3 to 6 carbon atoms; n represents an integer of from 1 to 4; and A represents $-COCH_3$ or $-C(OR^3)(OR^4)CH_3$ in which $R^3$ and $R^4$ independently represents an alkyl group having 1 to 3 carbon atoms or $R^3$ together with $R^4$ represents a polymethylene group having 2 to 4 carbon atoms; (b) a pharmacologically acceptable salt of the compound of formula (I) or (II); (c) a bronchodilating agent or a brain function improving agent comprising the compound of formula (I) or (II); and a pharmaceutical composition comprising the effective amount of the xanthine compound together with a pharmaceutically acceptable carrier or coating.

25. A method according to claim 24, wherein said step of administration is performed on a human being.